(19) Europäisches Patentamt
European Patent Office
Office européen des brevets

(11) **EP 4 417 625 A1**

(12) **EUROPEAN PATENT APPLICATION**
published in accordance with Art. 153(4) EPC

(43) Date of publication:
**21.08.2024 Bulletin 2024/34**

(21) Application number: **22880722.8**

(22) Date of filing: **16.09.2022**

(51) International Patent Classification (IPC):
**C07K 16/40** (2006.01)    **A61K 39/395** (2006.01)
**A61K 45/00** (2006.01)    **A61P 35/00** (2006.01)
**A61P 43/00** (2006.01)    **C12N 15/13** (2006.01)

(52) Cooperative Patent Classification (CPC):
**A61K 39/395; A61K 45/00; A61P 35/00;**
**A61P 43/00; C07K 16/00; C07K 16/40**

(86) International application number:
**PCT/JP2022/034801**

(87) International publication number:
**WO 2023/063026 (20.04.2023 Gazette 2023/16)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **15.10.2021 JP 2021169289**

(71) Applicant: **CHIOME BIOSCIENCE INC.**
**Shibuya-ku**
**Tokyo 151-0071 (JP)**

(72) Inventors:
• **TOYOURA, Masayoshi**
  **Tokyo 151-0071 (JP)**

• **KAMBAYASHI, Hiroaki**
  **Tokyo 151-0071 (JP)**
• **SAWADA, Atsushi**
  **Tokyo 151-0071 (JP)**
• **TAKESUE, Aki**
  **Tokyo 151-0071 (JP)**
• **INOUE, Toshikazu**
  **Tokyo 151-0071 (JP)**

(74) Representative: **HGF**
**HGF Europe LLP**
**Neumarkter Straße 18**
**81673 München (DE)**

Remarks:
The complete document including Reference Table(s) and the Sequence Listing(s) can be downloaded from the EPO website

(54) **ANTIBODY OR FRAGMENT THEREOF BINDING SPECIFICALLY TO MERTK, AND ANTI-TUMOR AGENT**

(57)    A problem addressed by the present invention is to provide an anti-MerTK antibody that has a tumor-suppressing effect and does not tend to cause ocular toxicity. The present invention provides an antibody or a fragment thereof that binds specifically to MerTK, in which the amino acid sequences of heavy chain and light chain CDRs 1 to 3 satisfy predetermined requirements.

FIG. 17

**Description**

TECHNICAL FIELD

The present invention relates to an antibody or a fragment thereof specifically binding to MerTK, and an anti-tumor agent.

BACKGROUND ART

"Myeloid-Epithelial-Reproductive Tyrosine Kinase" (hereinafter also referred to as "MerTK") is a known member of the TAM (Tyro3, AXL and MerTK) family of receptor tyrosine kinases. MerTK is a transmembrane protein having two immunoglobulin-like domains, two fibronectin type III domains and one tyrosine kinase domain.

MerTK is known to have various functions. For example, it has been reported that activation of MerTK expressed on cancer cells induces proliferation and migration of cancer cells and MerTK contributes to an immunosuppressive tumor microenvironment (e.g., Non-Patent Document 1 and Non-Patent Document 2). Because of this, MerTK now attracts attention as a novel target molecule in the cancer immunity field.

For example, Patent Document 1 indicates that an anti-MerTK antibody capable of inhibiting binding of MerTK with a ligand of MerTK "Growth arrest-specific protein 6" (hereinafter also referred to as "Gas6") is efficacious for cancer therapy.

On the other hand, inhibition of binding of MerTK with a ligand thereof may cause side effects. For example, Non-Patent Document 3 reports that administration of a MerTK small molecule inhibitor (UNC569) to mice caused increased toluidine blue-positive granules and vacuoles in photoreceptor, resulting in ocular toxicity. Non-Patent Document 4 reports that an anti-MerTK antibody having an efferocytosis inhibitory ability and anti-tumor effects was attributed to ocular toxicity in cynomolgus monkeys.

Citation List

Patent Document

Patent Document 1: PCT International Publication No. WO2020/076799

Non-Patent Document

Non-Patent Document 1: Pharmacol Ther. 2021 Sep;225:107822. doi: 10.1016/j.pharmthera.2021.107822. Epub 2021 Mar 10
Non-Patent Document 2: Immunity. 2020 Feb 18;52(2):357-373.e9. doi: 10.1016/j.immuni.2020.01.014. Epub 2020 Feb 11
Non-Patent Document 3: Toxicol Pathol. 2018 Feb;46(2):193-201. doi:10.1177/0192623317749469. Epub 2018 Jan 8
Non-Patent Document 4: https://www.surfaceoncolgy.com/wp-content/uploads/Surface_AACR19_190307c.pdf

DISCLOSURE OF THE INVENTION

Problems to be Solved by the Invention

However, to date, no reports have been found on an anti-MerTK antibody having tumor suppressive effects and hardly leading to ocular toxicity.

With the foregoing in view, an object of the present invention is to provide an anti-MerTK antibody having tumor suppressive effects and hardly leading to ocular toxicity. Means for Solving the Problems

The inventors of the present invention have found, as a result of extensive research, that an antibody or a fragment thereof having prescribed sequences can solve the problem, thereby achieving the present invention. The present invention specifically provides the following.

(1) An antibody or fragment thereof that specifically binds to MerTK and has heavy chain and light chain CDRs 1-3 having amino acid sequences fulfilling all requirements in [Requirements A] indicated below:

[Requirements A]

the heavy chain CDR1 has an amino acid sequence given in SEQ ID NO: 2;
the heavy chain CDR2 has an amino acid sequence given in SEQ ID NO: 3;
the heavy chain CDR3 has an amino acid sequence given in SEQ ID NO: 4;
the light chain CDR1 has an amino acid sequence given in SEQ ID NO: 6;
the light chain CDR2 has an amino acid sequence given in SEQ ID NO: 7; and
the light chain CDR3 has an amino acid sequence given in SEQ ID NO: 8.

(2) The antibody or fragment thereof according to (1), further fulfilling all requirements in [Requirements B] indicated below:
[Requirements B]

the heavy chain variable region has an amino acid sequence given in SEQ ID NO: 1; and
the light chain variable region has an amino acid sequence given in SEQ ID NO: 5.

(3) An antibody or fragment thereof that specifically binds to MerTK and has heavy chain and light chain CDRs 1-3 having amino acid sequences fulfilling all requirements in [Requirements C] indicated below:
[Requirements C]

the heavy chain CDR1 has an amino acid sequence given in SEQ ID NO: 10;
the heavy chain CDR2 has an amino acid sequence given in SEQ ID NO: 11;
the heavy chain CDR3 has an amino acid sequence given in SEQ ID NO: 12;
the light chain CDR1 has an amino acid sequence given in SEQ ID NO: 14;
the light chain CDR2 has an amino acid sequence given in SEQ ID NO: 15; and
the light chain CDR3 has an amino acid sequence given in SEQ ID NO: 16.

(4) The antibody or fragment thereof according to (3), further fulfilling all requirements in either [Requirements D] or [Requirements E] indicated below:

[Requirements D]

the heavy chain variable region has an amino acid sequence given in SEQ ID NO: 9; and
the light chain variable region has an amino acid sequence given in SEQ ID NO: 13;

[Requirements E]

the heavy chain variable region has an amino acid sequence given in SEQ ID NO: 23; and
the light chain variable region has an amino acid sequence given in SEQ ID NO: 24.

(5) An antibody or fragment thereof that specifically binds to MerTK and has heavy chain and light chain CDRs 1-3 having amino acid sequences fulfilling all requirements in [Requirements F] indicated below:
[Requirements F]

the heavy chain CDR1 has an amino acid sequence given in SEQ ID NO: 2;
the heavy chain CDR2 has an amino acid sequence given in SEQ ID NO: 18;
the heavy chain CDR3 has an amino acid sequence given in SEQ ID NO: 4;
the light chain CDR1 has an amino acid sequence given in SEQ ID NO: 6;
the light chain CDR2 has an amino acid sequence given in SEQ ID NO: 20; and
the light chain CDR3 has an amino acid sequence given in SEQ ID NO: 8.

(6) The antibody or fragment thereof according to (5), further fulfilling all requirements in either [Requirements G] or [Requirements H] indicated below:

[Requirements G]

the heavy chain variable region has an amino acid sequence given in SEQ ID NO: 17; and
the light chain variable region has an amino acid sequence given in SEQ ID NO: 19;

[Requirements H]

the heavy chain variable region has an amino acid sequence given in SEQ ID NO: 21; and
the light chain variable region has an amino acid sequence given in SEQ ID NO: 22.

(7) An anti-tumor agent, including the antibody or fragment thereof according to any one of (1) to (6).
(8) The anti-tumor agent according to (7), further including an immune checkpoint inhibitor.

Effects of the Invention

The present invention provides an anti-MerTK antibody having tumor suppressive effects and hardly leading to ocular toxicity.

BRIEF DESCRIPTION OF THE DRAWINGS

Fig. 1 shows results of the phosphorylation inhibition test in the Examples;
Fig. 2 shows amino acid sequences of the H chain variable region and the L chain variable region of the antibodies prepared in the Examples and positions of CDR mutations introduced to the sequences;
Fig. 3 shows reactivity of the antibodies prepared in the Examples in ELISA to MerTKs derived from various animals;
Fig. 4 shows tumor suppressive effects of the antibodies prepared in the Examples;
Fig. 5 shows tumor suppressive effects of the antibodies prepared in the Examples;
Fig. 6 shows M1/M2 ratio in the TIL analysis in the Examples; Fig. 7 shows tumor suppressive effects of the antibody prepared in the Examples;
Fig. 8 shows tumor suppressive effects of the antibody prepared in the Examples;
Fig. 9 shows tumor suppressive effects of the antibody prepared in the Examples;
Fig. 10 shows tumor suppressive effects of the antibody prepared in the Examples;
Fig. 11 shows tumor suppressive effects of the antibodies prepared in the Examples;
Fig. 12 shows tumor suppressive effects of the antibodies prepared in the Examples;
Fig. 13 shows ability of various anti-MerTK antibodies for inhibiting binding of MerTK with Gas6;
Fig. 14 shows ability of various anti-MerTK antibodies for inhibiting binding of MerTK with TULP1;
Fig. 15 shows ability of various anti-MerTK antibodies for inhibiting binding of MerTK with Gas6;
Fig. 16 shows ability of various anti-MerTK antibodies for inhibiting binding of MerTK with TULP1;
Fig. 17 shows results of TB staining of the retinal pigment epithelium after administration of various agents;
Fig. 18 shows results of TB staining of the outer nuclear layer after administration of various agents;
Fig. 19 shows results of TB staining of the retinal pigment epithelium after administration of various agents;
Fig. 20 shows results of TB staining of the outer nuclear layer after administration of various agents; and
Fig. 21 shows reactivity of human chimeric and humanized antibodies of an anti-MerTK antibody in ELISA.

PREFERRED MODE FOR CARRYING OUT THE INVENTION

The embodiments of the present invention will be described below, but the invention is not limited to these.

<Antibody or fragment thereof specifically binding to MerTK>

An antibody or fragment thereof (hereinafter also referred to as "antibody or fragment thereof of the present invention") specifically binding to MerTK according to the present invention includes three embodiments as described hereinbelow.

The antibody or fragment thereof of the present invention is an anti-MerTK antibody or fragment thereof. It has been conventionally known that anti-MerTK antibodies inhibit binding of MerTK with ligands of MerTK, thereby exhibiting anti-tumor effects. Anti-MerTK antibodies inhibit, for example, binding of MerTK with a ligand Gas6 of MerTK, resulting in modification of tumor microenvironments and exhibition of anti-tumor effects.

Among ligands of MerTK, "Tubby-related protein 1" (hereinafter also referred to as "TULP1") gene is a cause of an eye disease, retinitis pigmentosa. Further, it is known that knocking out of TULP1 causes photoreceptor degeneration (Investigative Ophthalmology & Visual Science, August 2001, Vol. 42, No. 9). It is also known that knocking out of MerTK shows shrinkage of the outer nuclear layer which is one of abnormalities known to correlate with deterioration of eyesight (Neuron, 2012 Dec 20; 76(6): 1123-32). Thus, anti-MerTK antibodies exhibiting anti-tumor effects which inhibit binding of MerTK with TULP1 may lead to ocular toxicity.

The inventors of the present invention successfully created, as a result of further exhaustive research, the antibody or fragment thereof of the present invention and have confirmed that although the antibody or the like inhibits binding of MerTK with Gas6, the antibody or the like does not inhibit binding of MerTK with TULP1, has tumor suppressive effects and hardly leads to ocular toxicity.

The phrase "specifically binds to MerTK" or the like as used herein means that the antibody or fragment thereof of the present invention specifically binds to MerTK protein and does not bind to or hardly binds to other proteins. Whether an antibody or fragment thereof specifically binds to MerTK is identified by enzyme-linked immunosorbent assay (ELISA).

The term "antibody" as used therein means a protein having a Y-shaped four-chain structure also referred to as immunoglobulin or the like.

The term "fragment of the antibody" as used herein means a protein having a structure of, among structures of the antibody of the present invention, a complementarity determining region (hereinafter also referred to as "CDR") or a variable region of the antibody. However, the fragment of the antibody of the present invention is an antigen-binding fragment that specifically binds to MerTK and fulfills at least all requirements in any of requirements A to C described hereinbelow.

(Antibody or fragment thereof according to the first embodiment)

Among the antibodies or fragments thereof of the present invention, an antibody or fragment thereof according to the first embodiment has heavy chain and light chain CDRs 1-3 that have amino acid sequences fulfilling all requirements in [Requirements A] indicated below:

[Requirements A]

the heavy chain CDR1 has an amino acid sequence given in SEQ ID NO: 2;
the heavy chain CDR2 has an amino acid sequence given in SEQ ID NO: 3;
the heavy chain CDR3 has an amino acid sequence given in SEQ ID NO: 4;
the light chain CDR1 has an amino acid sequence given in SEQ ID NO: 6;
the light chain CDR2 has an amino acid sequence given in SEQ ID NO: 7; and
the light chain CDR3 has an amino acid sequence given in SEQ ID NO: 8.

[Table 1]

| SEQ ID NO: | Amino acid sequence |
| --- | --- |
| 2 | SYWMH |
| 3 | NINPSNGGTNYNEKFKS |
| 4 | LNYYGFDGFAF |
| 6 | KASQDINSYLS |
| 7 | RANRLVD |
| 8 | LQYDEFPFT |

The antibody or fragment thereof according to the first embodiment preferably fulfills all requirements in [Requirements B] indicated below from the viewpoint that the effects of the present invention are more likely to be exhibited:

[Requirements B]

the heavy chain variable region has an amino acid sequence given in SEQ ID NO: 1; and
the light chain variable region has an amino acid sequence given in SEQ ID NO: 5.

[Table 2]

| SEQ ID NO: | Amino acid sequence |
|---|---|
| 1 | QVQLQQPGTELVKPGASVKLSCRASGNTFTSYWMHWVKQRPGQGLEWIGNIN PSNGGTNYNEKFKSKATLTVDKSSSTAYMQLSSLTSEDSAVYYCARLNYYGFD GFAFWGQGTLVTVSA |
| 5 | DIKMTQSPSSMYASLGERVTITCKASQDINSYLSWFQQKPGKSPKTLIYRANRL VDGVPSRFSGSGSGQDYSLTISSLEYEDMGIYYCLQYDEFPFTFGSGTKLEIK |

The antibody or fragment thereof according to the first embodiment includes antibodies referred to as "20A77", "20A77_mIgG1" and "20A77_mIgG2a" in the Examples.

(Antibody or fragment thereof according to the second embodiment)

Among the antibodies or fragments thereof of the present invention, an antibody or fragment thereof according to the second embodiment has heavy chain and light chain CDRs 1-3 that have amino acid sequences fulfilling all requirements in [Requirements C] indicated below:
[Requirements C]

the heavy chain CDR1 has an amino acid sequence given in SEQ ID NO: 10;
the heavy chain CDR2 has an amino acid sequence given in SEQ ID NO: 11;
the heavy chain CDR3 has an amino acid sequence given in SEQ ID NO: 12;
the light chain CDR1 has an amino acid sequence given in SEQ ID NO: 14;
the light chain CDR2 has an amino acid sequence given in SEQ ID NO: 15; and
the light chain CDR3 has an amino acid sequence given in SEQ ID NO: 16.

[Table 3]

| SEQ ID NO: | Amino acid sequence |
|---|---|
| 10 | VYDMH |
| 11 | RIRSKSSNYATNYADSVKD |
| 12 | DGSHYYYALDY |
| 14 | RASQDINNYLN |
| 15 | YSSRLHS |
| 16 | QQGKMFPWT |

The antibody or fragment thereof according to the second embodiment preferably fulfills all requirements in either [Requirements D] or [Requirements E] indicated below from the viewpoint that the effects of the present invention are more likely to be exhibited:

[Requirements D]

the heavy chain variable region has an amino acid sequence given in SEQ ID NO: 9; and
the light chain variable region has an amino acid sequence given in SEQ ID NO: 13;

[Requirements E]

the heavy chain variable region has an amino acid sequence given in SEQ ID NO: 23; and
the light chain variable region has an amino acid sequence given in SEQ ID NO: 24.

[Table 4]

| SEQ ID NO: | Amino acid sequence |
|---|---|
| 9 | EVQLVESGGGLVQPKGSLKLSCAASGFTFNVYDMHWVRQAPGEGLEWVARIR SKSSNYATNYADSVKDRFTISRDDSQSMLYLQMNNLQTEDTAIYYCVRDGSHY YYALDYWGQGTSVTVSS |
| 13 | DIQMTQTTSSLSASLGDRVTISCRASQDINNYLNWYQQKLDGTVKLLIYYSSRL HSGVPSRFSGSGSGTDYSLTISNLEQEDIATYFCQQGKMFPWTFGGGTKLEIK |
| 23 | EVQLVESGGGLVQPGGSLRLSCAASGFTFNVYDMHWVRQAPGKGLEWVARIR SKSSNYATNYADSVKDRFTISRDDAKNSLYLQMNSLRAEDTAVYYCVRDGSHY YYALDYWGQGTTVTVSS |
| 24 | DIQMTQSPSSLSASVGDRVTITCRASQDINNYLNWYQQKPGKAPKLLIYYSSRL HSGVPSRFSGSGSGTDYTLTISSLQPEDFATYYCQQGKMFPWTFGGGTKVEIK |

The antibody or fragment thereof according to the second embodiment includes antibodies referred to as "24A1", "24A1_mIgG1", "24A1_mIgG2a", "24A1_hIgG1LALA", "hz24A1" and "hz24A1_hIgGILALA" in the Examples. "hz24A1" and "hz24A1_hIgG1LALA" are both humanized antibodies derived from "24A1".

(Antibody or fragment thereof according to the third embodiment)

Among the antibodies or fragments thereof of the present invention, an antibody or fragment thereof according to the third embodiment has heavy chain and light chain CDRs 1-3 that have amino acid sequences fulfilling all requirements in [Requirements F] indicated below:

[Requirements F]

the heavy chain CDR1 has an amino acid sequence given in SEQ ID NO: 2;
the heavy chain CDR2 has an amino acid sequence given in SEQ ID NO: 18;
the heavy chain CDR3 has an amino acid sequence given in SEQ ID NO: 4;
the light chain CDR1 has an amino acid sequence given in SEQ ID NO: 6;
the light chain CDR2 has an amino acid sequence given in SEQ ID NO: 20; and
the light chain CDR3 has an amino acid sequence given in SEQ ID NO: 8.

[Table 5]

| SEQ ID NO: | Amino acid sequence |
|---|---|
| 2 | SYWMH |
| 18 | NINPSSGGTNYNEKFKS |
| 4 | LNYYGFDGFAF |
| 6 | KASQDINSYLS |
| 20 | RANRLVS |
| 8 | LQYDEFPFT |

The antibody or fragment thereof according to the third embodiment preferably fulfills all requirements in either [Requirements G] or [Requirements H] indicated below from the viewpoint that the effects of the present invention are more likely to be exhibited:

[Requirements G]

the heavy chain variable region has an amino acid sequence given in SEQ ID NO: 17; and
the light chain variable region has an amino acid sequence given in SEQ ID NO: 19;

[Requirements H]

the heavy chain variable region has an amino acid sequence given in SEQ ID NO: 21; and
the light chain variable region has an amino acid sequence given in SEQ ID NO: 22.

[Table 6]

| SEQ ID NO: | Amino acid sequence |
|---|---|
| 17 | QVQLQQPGTELVKPGASVKLSCRASGNTFTSYWMHWVKQRPGQGLEWIGNIN PSSGGTNYNEKFKSKATLTVDKSSSTAYMQLSSLTSEDSAVYYCARLNYYGFD GFAFWGQGTLVTVSA |
| 19 | DIKMTQSPSSMYASLGERVTITCKASQDINSYLSWFQQKPGKSPKTLIYRANRL VSGVPSRFSGSGSGQDYSLTISSLEYEDMGIYYCLQYDEFPFTFGSGTKLEIK |
| 21 | QVQLVQSGAEVKKPGSSVKVSCKASGNTFTSYWMHWVRQAPGQGLEWIGNIN PSSGGTNYNEKFKSRVTITVDKSTSTAYMELSSLRSEDTAVYYCARLNYYGFD GFAFWGQGTLVTVSS |
| 22 | DIQMTQSPSSLSASVGDRVTITCKASQDINSYLSWFQQKPGKAPKTLIYRANRL VSGVPSRFSGSGSGTDYTLTISSLQPEDFATYYCLQYDEFPFTFGGGTKVEIK |

The antibody or fragment thereof according to the third embodiment includes antibodies referred to as "m20A77", "m20A77_mIgG1", "m20A77_hIgG1LALA", "hz20A77" and "hz20A77_hIgG1LALA" in the Examples. "hz20A77" and "hz20A77_hIgG1LALA" are both humanized antibodies derived from "m20A77".

<Method for producing the antibody or fragment thereof of the present invention>

Producing method of the antibody or fragment thereof of the present invention is not limited, and any conventionally known methods for producing various antibodies (such as monoclonal antibodies and polyclonal antibodies) and various polypeptides can be used.
The antibody of the present invention is preferably produced as a monoclonal antibody.
The antibody or fragment thereof of the present invention may be purified, if necessary.
Whether or not an antibody or a fragment thereof is the antibody or fragment thereof of the present invention can be identified by any methods (such as N-terminal amino acid sequencing) for identifying amino acid sequences.
The present invention encompasses a gene encoding the antibody or fragment thereof of the present invention, an expression vector including the gene and the like. The gene and expression vector can be prepared according to conventionally known methods.

<Anti-tumor agent of the present invention>

The antibody or fragment thereof of the present invention exhibits preferable anti-tumor effects, and thus can be preferably used as an anti-tumor agent.
The anti-tumor agent of the present invention may be efficacious for therapy of, for example, cancer (such as lung cancer, breast cancer, colon cancer and acute lymphoblastic leukemia).

The anti-tumor agent of the present invention can be administered according to methods similar to those for conventionally known antibody drugs. For example, the anti-tumor agent of the present invention can be administered by oral, intravenous and subcutaneous administrations.

Any amount of the antibody or fragment thereof of the present invention without limitation may be included in the anti-tumor agent of the present invention. The amount can be set, as appropriate, according to the condition of a subject, desired effects and the like.

The anti-tumor agent of the present invention may be administered to any subjects without limitation. Examples of the subject include mammals (such as humans and non-human animals). Examples of the non-human animals include monkeys.

The anti-tumor agent of the present invention may contain an arbitrary component that is other than the antibody or fragment thereof of the present invention and is a known ingredient for medicaments in a range that does not inhibit the effects of the present invention. The amount and type of such component can be set, as appropriate, according to desired effects and the like.

The anti-tumor agent of the present invention may or may not contain an active component other than the antibody or fragment thereof of the present invention. The anti-tumor agent of the present invention may be used in combination with an active component other than the antibody or fragment thereof of the present invention. Such an active component may be administered as a single drug containing the same together with the antibody or fragment thereof of the present invention, or may be prepared as a drug separate from the antibody or fragment thereof of the present invention and may be administered in combination with the anti-tumor agent of the present invention.

Examples of preferable active components that may be used in combination with the antibody or fragment thereof of the present invention include immune checkpoint inhibitors. Examples of the immune checkpoint inhibitor include anti-PD-1 antibodies, anti-PD-L1 antibodies and anti-CTLA4 antibodies.

The present invention encompasses a method for treating cancer using the anti-tumor agent of the present invention.

EXAMPLES

The present invention will be further specifically described hereinbelow by way of Examples. However, the present invention is not limited to those Examples.

<Test 1: Establishment of monoclonal antibodies for MerTK>

Monoclonal antibodies for MerTK were obtained on the basis of the method indicated below.

(1-1) Construction of human MerTK expression vector

On the basis of the amino acid sequence (UniProt No: Q12866) of human MerTK protein, DNA containing the Kozak translation initiation sequence at the 5'end and a translation termination codon at the 3' side inserted therein was synthesized (using GENEWIZ gene synthesis service). Using "In-Fusion HD Cloning Kit" (Takara Bio Inc.), the synthesized DNA was inserted to the cloning site of "pcDNA 3.4" (Thermo Fisher Scientific Inc.) to obtain a human MerTK expression vector.

(1-2) Construction of mouse MerTK expression vector

On the basis of the amino acid sequence (UniProt No: Q60805) of mouse MerTK protein, a mouse MerTK expression vector was obtained according to the same method as the human MerTK expression vector above.

(2-1) Expression and purification of human MerTK-mouse Fc fusion protein

As a human MerTK-mouse Fc fusion protein (hereinafter also referred to as "hMerTKECD-mFc"), a protein containing amino acids in positions 1-505 of human MerTK protein (UniProt No: Q12866) fused to amino acids in positions 114-330 of mouse IgG2a heavy chain (UniProt No: P01863) was designed. On the basis of the amino acid sequence, an expression vector was constructed, resulting in a hMerTKECD-mFc expression vector. Next, by using the hMerTKECD-mFc expression vector as a plasmid to be introduced, transient expression was performed using "Expi293 expression system" (Thermo Fisher Scientific Inc.). After the transient expression, a culture supernatant was recovered and subjected to affinity purification using "HiTrap Protein G HP column" (Cytiva). The eluent of the affinity purification was concentrated and subjected to gel filtration purification using "Sephadex 200pg 16/60" (Cytiva). Elution fractions of the gel filtration purification were subjected to SDS-PAGE and fractions with observed bands were collected and used as hMerTKECD-mFc.

(2-2) Expression and purification of mouse MerTK-mouse Fc fusion protein

As a mouse MerTK-mouse Fc fusion protein (hereinafter also referred to as "mMerTKECD-mFc"), a protein containing amino acids in positions 1-497 of mouse MerTK protein (UniProt No: Q60805) fused to amino acids in positions 114-330 of mouse IgG2a heavy chain (UniProt No: P01863) was designed. On the basis of the amino acid sequence, the protein was expressed and purified according to the same method as the method for preparing hMerTKECD-mFc above, thereby obtaining mMerTKECD-mFc.

(3-1) Construction of EL4 cells stably expressing human MerTK

The human MerTK expression vector as a plasmid was transferred to a mouse lymphoma cell line EL4 using "Nucleofector" (Lonza). After the gene transfer, "G418" (Nacalai Tesque, Inc.) was added to a medium, and the cells were plated in a 96-well plate and cultured. After the cultivation, cells exhibited G418-resistant growth were subjected to flow cytometry using an anti-human MerTK antibody (R&D Systems, MAB8912) to verify expression of human MerTK. A monoclone-derived cell line was ultimately selected and used for the tests indicated below as EL4 cells stably expressing human MerTK (hereinafter also referred to as "hMerTK-EL4").

(3-2) Construction of BaF/3 cells stably expressing mouse MerTK

The mouse MerTK expression vector as a plasmid was transferred to a mouse pro-B cell line Ba/F3 using "Nucleofector" (Lonza). After the gene transfer, "G418" (Nacalai Tesque, Inc.) was added to a medium, and the cells were plated in a 96-well plate and cultured. After the cultivation, cells exhibited G418-resistant growth were subjected to flow cytometry using an anti-mouse MerTK antibody (R&D Systems, AF591) to verify expression of mouse MerTK. A monoclone-derived cell line was ultimately selected and used for the tests indicated below as BaF/3 cells stably expressing mouse MerTK (hereinafter also referred to as "mMerTK-Ba/F3").

(4) Preparation of monoclonal antibodies for MerTK

On the basis of the following two methods, MerTK purified proteins or cells stably expressing MerTK were used to immunize MerTK knockout mice (The Jackson Laboratory, B6;129-MerTK<tm1G41>/J). After the immunization, isolated lymph node cells were used to prepare monoclonal antibodies for MerTK.

(Immunization method-1)

hMerTKECD-mFc and mMerTKECD-mFc (both MerTK purified proteins) were used as antigens. The antigens were respectively mixed with an adjuvant "TiterMax Gold" (TiterMax) and intravenously administered to mouse footpad so that the antigen dose per mouse was 100 μg. After 7 and 10 days of the administration, animals were boosted according to the same manner as above except that the antigen dose per mouse was 10 μg. After completion of the immunization, the popliteal lymph node was harvested from mice, a cell suspension was prepared, then mixed with SP2/0-Ag14 myeloma cells and subjected to electro cell fusion using an electro cell fusion device ("ECFG21", Nepa Gene Co., Ltd.). The fused cells were suspended in "ClonaCellTM-HY Medium D" ("ST-03804", Stemcell Technologies) and plated to a plastic dish. After the plating, colonies formed after 8-10 days were isolated in a 96-well plastic plate of which wells respectively containing a hybridoma medium, and the culture supernatants thereof were used for the following evaluation. The hybridoma medium used contained a 1/50 amount of "Nutridom-CS" ("11363743001", Merck) and a 1/50 amount of "HAT Supplement" ("21060017", Thermo Fisher Scientific Inc.) relative to RPMI1640 ("A1049101", Thermo Fisher Scientific Inc.).

(Immunization method-2)

hMerTK-EL4 and mMerTK-Ba/F3 (both cells stably expressing MerTK) were used as antigens. The cells were prepared as suspensions in PBS. The antigens were respectively mixed with an adjuvant "TiterMax Gold" (TiterMax) and intravenously administered to mouse footpad so that the antigen dose per mouse was $1 \times 10^7$ cells. After 7 and 10 days of the administration, animals were boosted according to the same manner as above except that the antigen dose per mouse was $1 \times 10^5$ cells and the administration was intraperitoneally carried out. After the immunization, culture supernatants were obtained according to the same manner as in "Immunization method-1) above and used for the following evaluation.

(5) Antibody screening

Using the culture supernatants obtained as above, hybridoma clones capable of binding to human and mouse MerTKs were screened. Specifically, antibody binding was evaluated by flow cytometry using a human melanoma cell line G361 intrinsically expressing human MerTK and mMerTK-Ba/F3. As a result of screening about 15,000 clones, 76 hybridoma clones capable of binding to human and mouse MerTKs were obtained.

<Test 2: Antibody evaluation>

The hybridoma clones obtained as above were subjected to the following respective evaluations and functions of monoclonal antibodies for MerTK were studied.

(1) Phosphorylation inhibitory activity

On the basis of the procedures indicated below, the clones were evaluated for MerTK phosphorylation inhibitory activity. Higher activity means higher binding ability to MerTK.

(Procedures)

The cells (hMerTK-EL4) stably expressing human MerTK were cultured in a "DMEM high glucose medium" (Thermo Fisher Scientific Inc.) containing 10% FBS. After the cultivation, the cells were washed in PBS, suspended in a serum-free medium, plated in a 96-well plate at $1 \times 10^6$ cells/well and incubated at 37°C for 3 hours. After the incubation, the respective clones were added, the hMerTK-EL4 was allowed to react for 1 hour, stimulated with 200 nM human Gas6 (R&D Systems) for 10 minutes and centrifuged and collected. The collected hMerTK-EL4 was lysed in a cell lysis solution, and the level of the phosphorylated MerTK protein in the lysate was determined using "Human Phospho-Mer DuoSet IC ELISA" (R&D Systems). The cell lysis solution used contained 1% "IGEPAL CA-630" (SIGMA) and $1 \times$ concentration of "Halt Protease and Phosphatase Inhibitor Single-Use Inhibitor Cocktail" (Thermo Fisher Scientific Inc.) added to MilliQ water.

(Results)

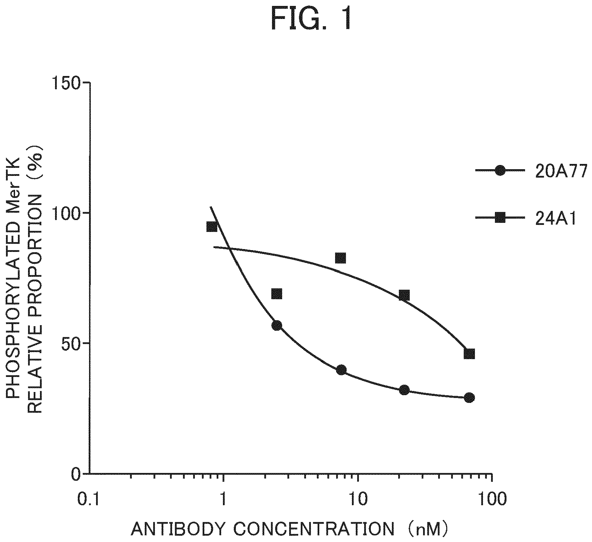

Among 76 clones, 14 clones showed phosphorylation inhibitory activity. Among these 14 clones, "20A77" and "24A1" were selected as clones showing particularly high inhibitory activity and subjected to the following tests. Fig. 1 shows the phosphorylation inhibitory activity of these clones. As shown in Fig. 1, it was found that "20A77" and "24A1" respectively inhibited binding of Gas6 with MerTK and MerTK phosphorylation in a concentration dependent manner and thus had high binding ability to MerTK.

(2) Antibody sequencing

On the basis of the following procedures, "20A77" and "24A1" were respectively sequenced for the immunoglobulin gene variable regions. The hybridoma cells were expanded, RNA was extracted using "RNeasy mini kit" (Qiagen) and cDNA was synthesized using "Superscript III First-Strand Synthesis Super mix" (Invitrogen). The synthesized cDNA was used as a template to amplify the sequences of antibody variable regions by PCR. The amplification was carried out with primers respectively recognizing upstream of the variable region and downstream of the constant region for both heavy and light chains. The obtained DNA fragments were cloned into the vector attached to "Zeroblunt TOPO TA Cloning and Sequencing kit" (Thermo Fisher Scientific Inc.) and sequenced. CDR regions were determined in the obtained antibody sequences according to the method of Kabat et al. (Sequences of Proteins of Immunological Interests, Fifth edition, NIH Publication No. 91-3242, U.S. Department of Health and Human Services, 1991).

The antibody sequences and sequences of the CDR regions thereof analyzed are summarized in Table 7 below. The abbreviations in Tables hereinbelow have the following meanings.

VH: Heavy chain variable region
CDRH: Heavy chain CDR
VL: Light chain variable region
CDRL: Light chain CDR

[Table 7]

|  | VH | CDRH 1 | CDRH 2 | CDRH 3 | VL | CDRL 1 | CDRL 2 | CDRL 3 |
|---|---|---|---|---|---|---|---|---|
| 20A77 | SEQ ID NO:1 | SEQ ID NO:2 | SEQ ID NO:3 | SEQ ID NO:4 | SEQ ID NO:5 | SEQ ID NO:6 | SEQ ID NO:7 | SEQ ID NO:8 |
| 24A1 | SEQ ID NO:9 | SEQ ID NO:10 | SEQ ID NO:11 | SEQ ID NO:12 | SEQ ID NO:13 | SEQ ID NO:14 | SEQ ID NO:15 | SEQ ID NO:16 |

(3) Preparation of recombinant mouse antibody and antibody sequence modification

The respective antibody sequences indicated in Table 7 were joined to the mouse IgG1 heavy chain and κ light chain or the mouse IgG2a heavy chain and κ light chain, thereby designing mouse IgG1 and mouse IgG2a antibody gene sequences. On the basis of the designed antibody sequences, expression vectors were constructed, resulting in antibody expression vectors. By using the antibody expression vectors as plasmids to be transferred, transient expression was performed using "Expi293 expression system" (Thermo Fisher Scientific Inc.). The culture supernatants of transient expression were recovered and subjected to affinity purification using "Ab-Capcher ExTra column" (ProteNova Co., Ltd.). The eluent of the affinity purification was concentrated and subjected to gel filtration purification using "Sephadex 200pg 16/60" (Cytiva) with histidine buffer (1% Histidine, pH 6.0). The elution fractions of the gel filtration purification were subjected to SDS-PAGE and bands were observed. The fractions for which bands were observed were collected to obtain mouse IgG1 antibodies and mouse IgG2a antibodies for "20A77" and "24A1" (respectively also referred to as "20A77_mIgG1", "20A77_mIgG2a", "24A1_mIgG1" and "24A1_mIgG2a").

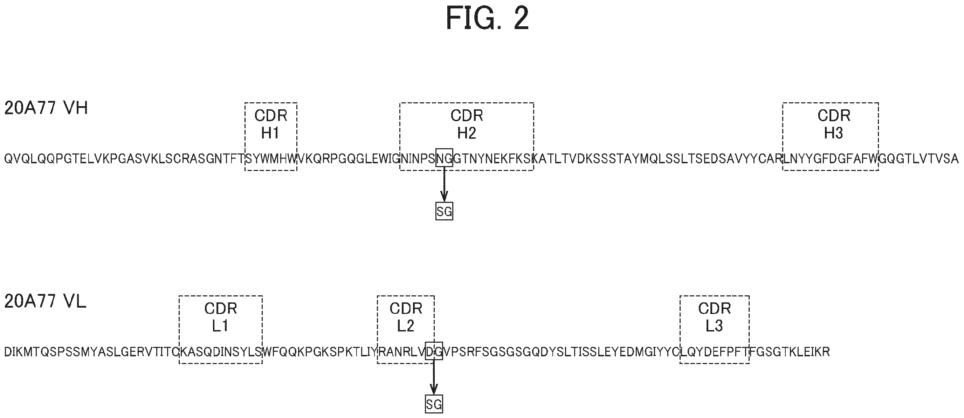

Further, modification of amino acids in the CDR regions was sought. Fig. 2 shows the positions where the sequence modifications were introduced, and the modifications were made in CDR region sequences which highly possibly undergo cleavage and deamination reactions. The antibody provided with the CDR sequence modifications is referred to as "m20A77". Table 8 below shows sequence information thereof. For "m20A77", a mouse IgG1 antibody (also referred to as "m20A77_mIgG1") was obtained according to the same manner as above.

[Table 8]

|  | VH | CDRH 1 | CDRH 2 | CDRH 3 | VL | CDRL 1 | CDRL 2 | CDRL 3 |
|---|---|---|---|---|---|---|---|---|
| m20A77 | SEQ ID NO:17 | SEQ ID NO:2 | SEQ ID NO:18 | SEQ ID NO:4 | SEQ ID NO:19 | SEQ ID NO:6 | SEQ ID NO:20 | SEQ ID NO:8 |

(4) Cross-reactivity between MerTKs from various animals

The antibody clones obtained as above were studied for cross-reactivity between MerTKs derived from various animals (humans, mice, cynomolgus monkeys and rats) by ELISA. In the ELISA, the antigens used were human Fc fusion proteins of MerTKs derived from various animals (humans, mice, cynomolgus monkeys and rats). Specifically, human and mouse MerTK antigens used were "Recombinant Human Mer Fc Chimera Protein" (R&D systems) and "Recombinant Mouse Mer Fc Chimera Protein" (R&D systems). Cynomolgus MerTK and rat MerTK antigens used were cynomolgus MerTKECD-hFc (hereinafter also referred to as "cynoMerTKECD-hFc") and rat MerTKECD-hFc (hereinafter also referred to as "ratMerTKECD-hFc") prepared in the same manner as that for hMerTKECD-mFc and mMerTKECD-mFc.

The ELISA was performed according to the procedures indicated below. First, the respective antigens were added to an ELISA plate (Nunc) at a concentration of 5 μg/ml and immobilized overnight at room temperature. After removing the immobilization solution, 1% BSA/PBS was added for blocking reaction and left to stand at room temperature for 1 hour. After the blocking, the plate was washed with 0.05% Tween 20/PBS and an antibody solution was added for primary reaction at room temperature for 1 hour. After washing, an HRP-labelled antibody (GE Healthcare) was added for secondary reaction for 1 hour. After washing, a chromogenic substrate solution of TMB (Dako) was added for chromogenic reaction for 10 minutes, 1 N sulfuric acid was added to terminate the reaction and the absorbance was measured at 450 nm.

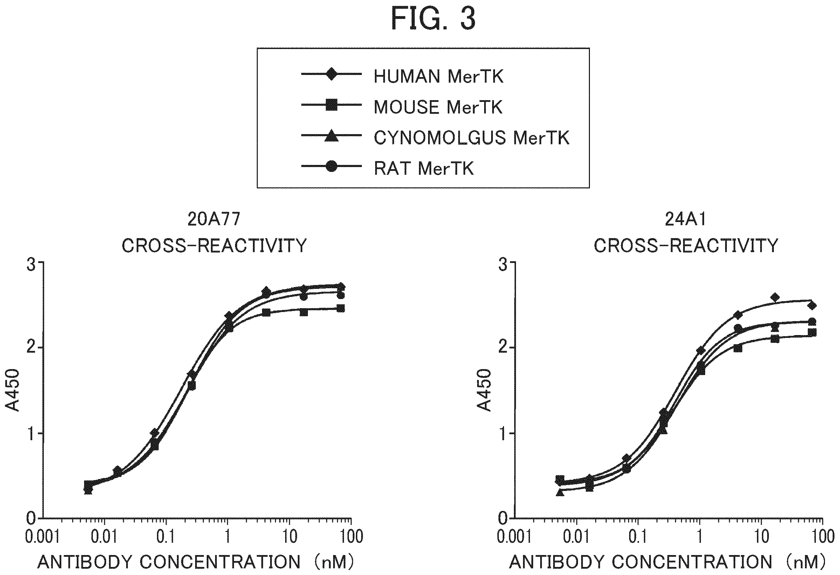

Fig. 3 shows reactivity of "m20A77_mIgG1" and "m24A1_mIgG1" in ELISA to MerTKs derived from various animals. As shown in Fig. 3, both "20A77" and "24A1" showed similar reactivity to human, mouse, cynomolgus monkey and rat antigens. These results show that both "20A77" and "24A1" have high species cross-reactivity.

<Test 3: anti-tumor effects of the antibody of the present invention>

On the basis of the procedures indicated below, anti-tumor effects of "20A77_mIgG2a" was evaluated. A syngeneic tumor model using the mouse colon cancer cell line CT26 (ATCC) was used for the evaluation.
CT26 cells ($5 \times 10^6$ cells per mouse) were subcutaneously transplanted to Balb/c mice at the right flank. After 5 days of the tumor transplantation, the mice were divided into groups and an isotype control antibody mIgG2a and "20A77_mIgG2a" were respectively intraperitoneally administered at a dose of 10 mg/kg at a frequency of twice a week. After the administration, the tumor size was measured at a frequency of twice a week until day 19 after the tumor transplantation. The tumor size was calculated from the equation below with the minor axis and the major axis of the tumor measured using calipers:

$$\text{Tumor size} = (\text{Minor axis (mm)})^2 \times (\text{Major axis (mm)}) \times 3.14/6$$

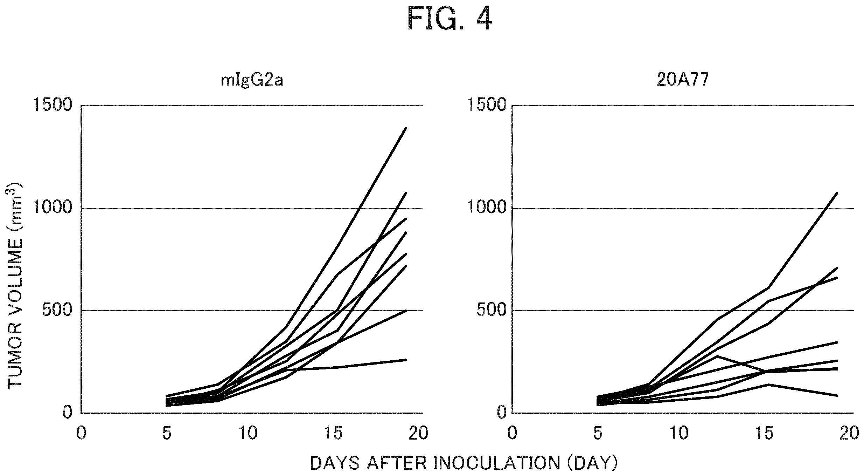

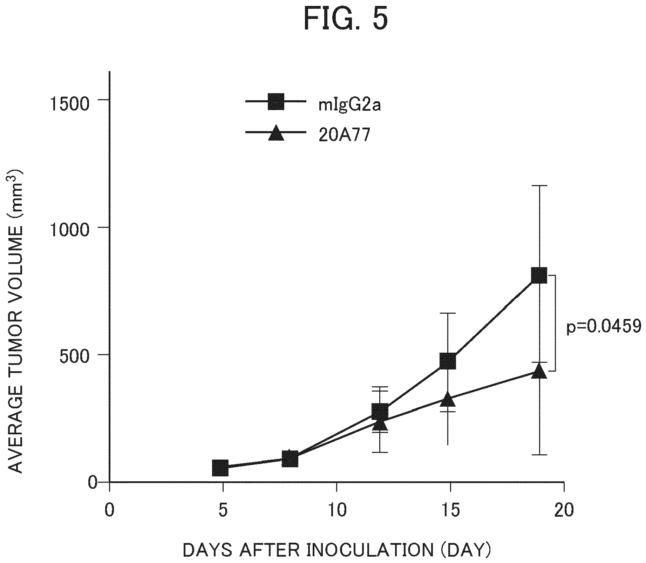

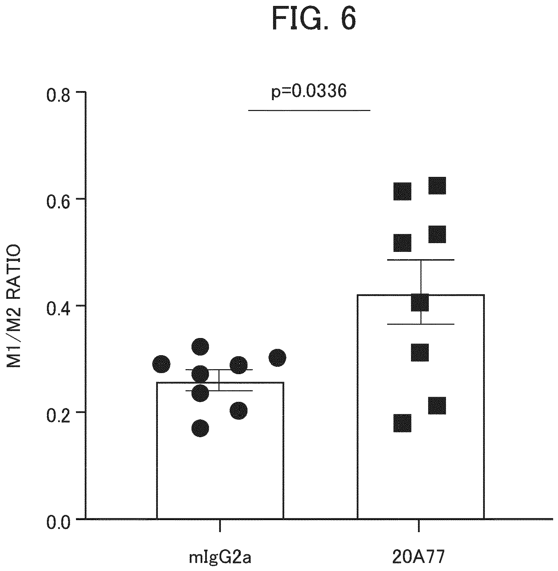

Figs. 4 and 5 show the evaluation results. As can be seen from the results, "20A77_mIgG2a" significantly suppressed tumor compared to the mIgG2a administration group.
Further, the tumor was collected on day 19 after tumor transplantation and subjected to tumor-infiltrating lymphocyte (TIL) analysis. For the TIL analysis, after collection of the tumor, the tumor mass was added to "gentleMACS C Tubes" (Miltenyi Biotec) together with an enzyme solution of "Tumor Dissociation kit, mouse" (Miltenyi Biotec) and the tumor was dissociated using "gentleMACS Octo Dissociator" (Miltenyi Biotec). After the tumor was dissociated, the tumor was washed with 1% FBS/PBS and then stained using the flow cytometry (FCM) analysis antibodies indicated in Table 9. After the staining, the FCM analysis was carried out using FACS calibur (BD). Fig. 6 shows the "M1/M2 ratio". M1 represents inflammatory macrophages and M2 represents immunosuppressive macrophages. The M1/M2 ratio is an index indicating a change characteristic to tumor microenvironments due to MerTK inhibition. Fig. 6 shows that administration of "20A77_mIgG2a" increases the M1/M2 ratio and the tumor microenvironment is transformed into an antitumor environment.

[Table 9]

| Antibody | Manufacturer | Catalog No. |
|---|---|---|
| FITC anti- mouse CD45 Antibody | Biolegend | 103108 |
| APC anti- mouse F4/80 Antibody | Biolegend | 123115 |
| FITC anti- mouse CD86 Antibody | Biolegend | 105005 |
| PE anti- mouse CD206 (MMR) Antibody | Biolegend | 141705 |

<Test 4: Efficacious dosage of the antibody of the present invention>

On the basis of the procedures indicated below, anti-tumor effects of "20A77_mIgG1" and "24A1_mIgG1" according to the dosage thereof were evaluated. As "Test 3" above, a syngeneic tumor model using the mouse colon cancer cell line CT26 (ATCC) was used for the evaluation.
In the "20A77_mIgG1" administration group, a solvent (1% histidine buffer, pH 6.0) and "20A77_mIgG1" were intraperitoneally administered at a dose of 3, 10 or 30 mg/kg at a frequency of twice a week. In the "24A1_mIgG1" administration group, a solvent (1% histidine buffer, pH 6.0) and "24A1_mIgG1" were intraperitoneally administered at a dose of 3, 5 or 30 mg/kg at a frequency of twice a week. The tumor size was measured at a frequency of twice a week until day 19 and 22 after the tumor transplantation for the "20A77_mIgG1" and "24A1_mIgG1" administration groups, respectively.

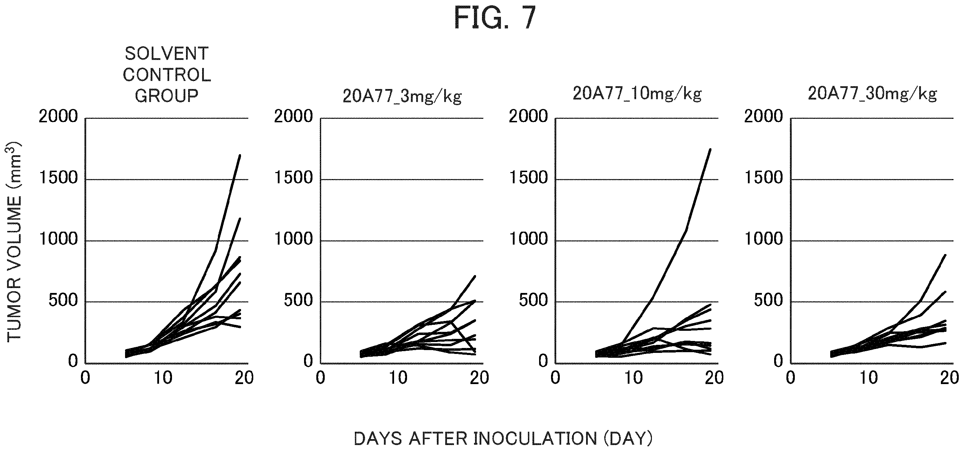

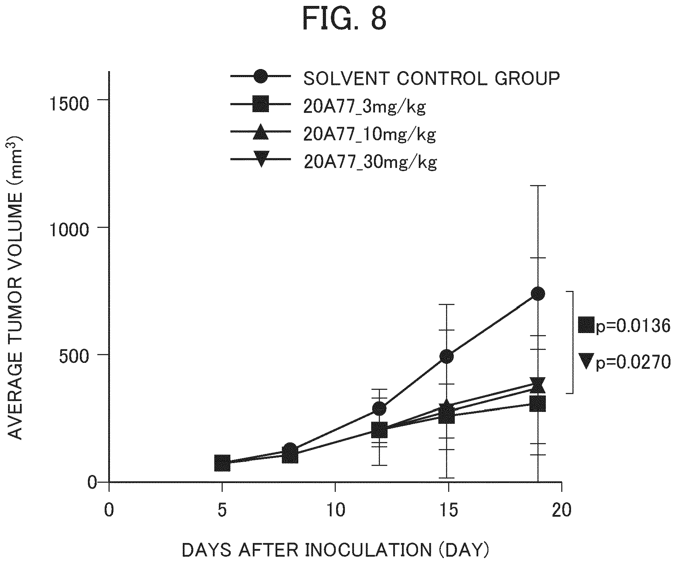

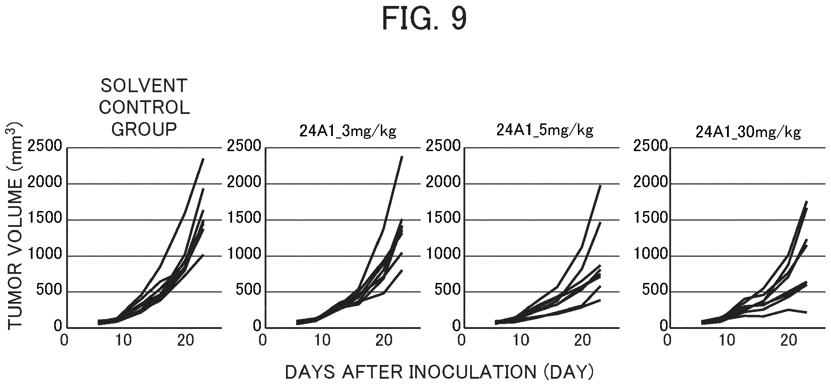

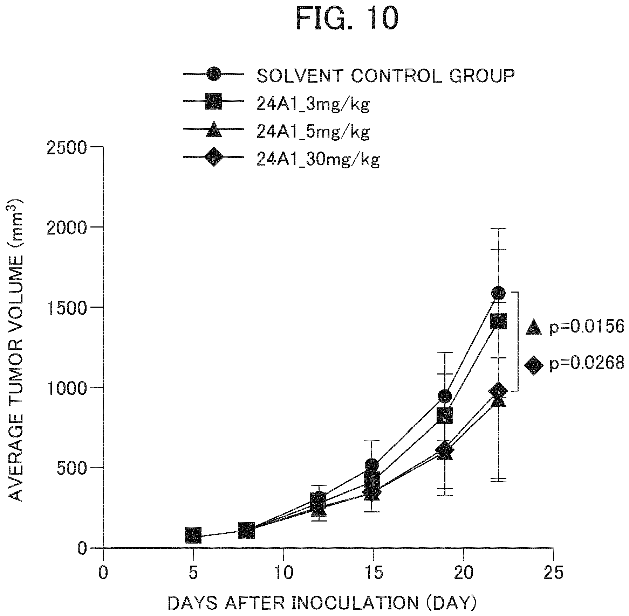

Figs. 7 to 10 show the evaluation results. As shown in Figs. 7 and 8, the "20A77_mIgG1" administration group showed tumor growth suppressing effects compared to the solvent control group at all concentrations. Particularly, significant suppressing effects were observed in the 3 mg/kg and 30 mg/kg administration groups. As shown in Figs. 9 and 10, the "24A1_mIgG1" administration group showed significant tumor growth suppression particularly in the 5 mg/kg and 30 mg/kg administration groups compared to the solvent control group.

<Test 5: anti-tumor effects of combined administration of the antibody of the present invention and an anti-PD-1 antibody>

On the basis of the procedures indicated below, anti-tumor effects of combined use of each of "20A77_mIgG1" and "24A1_mIgG1" and an anti-PD-1 antibody were evaluated. As "Test 3" above, a syngeneic tumor model using the mouse colon cancer cell line CT26 (ATCC) was used for the evaluation. Anti-PD-1 antibodies are known to have anti-tumor effects.

The administration groups received the antibodies indicated below at a frequency of twice a week. The administration was continued until day 19 after the tumor transplantation.

"mIgG1": "mIgG1" at 5 mg/kg
"m20A77": "20A77_mIgG1" at 3 mg/kg
"24A1": "24A1_mIgG1" at 5 mg/kg
"αPD-1": Anti-mouse PD-1 antibody ("29F.1A12", BioXcell) at 0.3 mg/kg
"m20A77+αPD-1": "m20A77_mIgG1" and the anti-mouse PD-1 antibody at 3 mg/kg and 0.3 mg/kg, respectively
"24A1+αPD-1": "24A1_mIgG1" and the anti-mouse PD-1 antibody at 5 mg/kg and 0.3 mg/kg, respectively

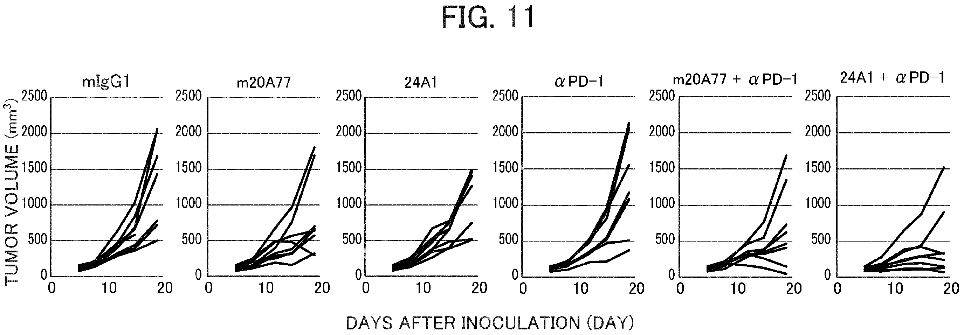

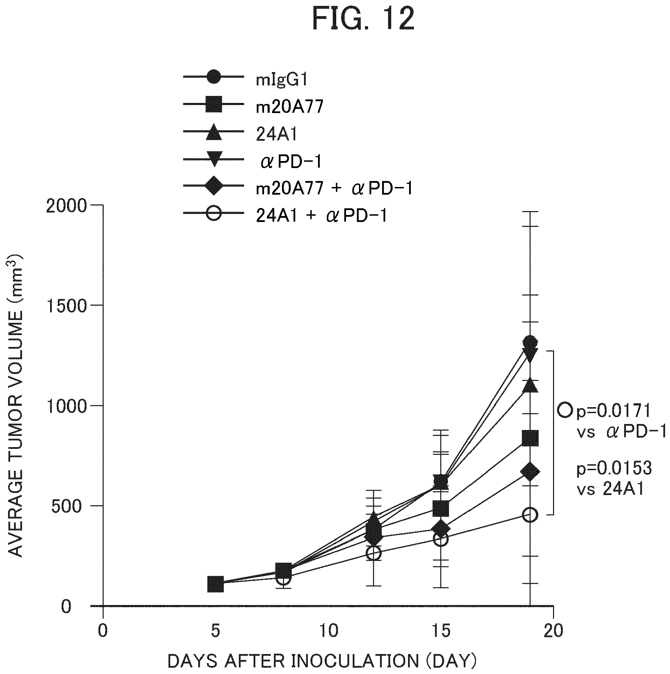

Figs. 11 and 12 show the evaluation results. As can be seen from these figures, "20A77_mIgG1" tended to have enhanced anti-tumor effects as a result of combined use with the PD-1 antibody. "24A1_mIgG1" had enhanced anti-tumor effects as a result of combined use with the PD-1 antibody. Consequently, it was found that the antibody of the present invention has enhanced anti-tumor effects as a result of combined use thereof with an anti-PD-1 antibody.

<Test 6: Evaluation of various ligand binding inhibitory ability>

"20A77" and "24A1" were evaluated for the ability to inhibit binding of MerTK with various ligands. The MerTK ligands used were human Gas6 (R&D Systems) and human TULP1 (abcam plc.) which were respectively biotinylated using "Biotin Labeling Kit-NH" (Dojindo Laboratories Co., Ltd.). Gas6 is a ligand correlated with anti-tumor effects, and an antibody having an activity to inhibit binding of MerTK with Gas6 may exhibit anti-tumor effects. TULP1 is a ligand correlated with ocular toxicity, and an antibody having an activity to inhibit binding of MerTK with TULP1 may have ocular toxicity. Thus, an antibody having high inhibitory activity of binding to Gas6 and low inhibitory activity of binding to TULP1 may be an efficacious anti-tumor agent having low ocular toxicity.

The ligand binding inhibition test was carried out as indicated below. First, human MerTK (R&D Systems) was added to an ELISA plate (Nunc) at a concentration of 5 μg/ml and immobilized overnight at room temperature. After removing the immobilization solution, 1% BSA/PBS was added for blocking reaction and left to stand at room temperature for 1 hour. After the blocking, the plate was washed with 0.05% Tween 20/PBS and an antibody solution was added for primary reaction at room temperature for 1 hour. After washing, biotinylated Gas6 or biotinylated TULP1 was added at a concentration of 10 nM or 40 nM, respectively, for secondary reaction at room temperature for 1 hour. After washing, a HRP-labelled streptavidin (Thermo Fisher Scientific Inc.) was added for tertiary reaction for 30 minutes. After washing, a chromogenic substrate solution of TMB (Dako) was added for chromogenic reaction for 20 minutes, 1 N sulfuric acid was added to terminate the reaction and the absorbance was measured at 450 nm.

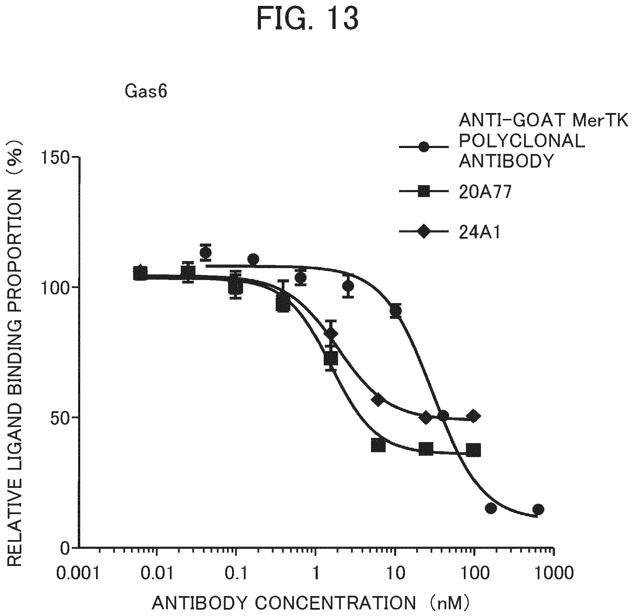

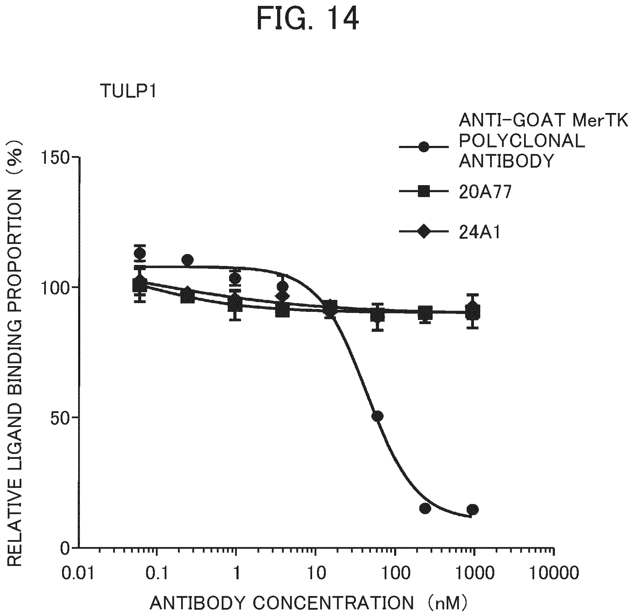

Figs. 13 and 14 show the evaluation results. As shown in these figures, the positive control (anti-goat MerTK polyclonal antibody) inhibited binding of both Gas6 and TULP1 with MerTK. On the other hand, although "20A77" and "24A1" inhibited binding of Gas6 with MerTK, both antibodies did not inhibit binding of TULP1 with MerTK. The above results show that the antibodies of the present invention may be antibodies having low ocular toxicity while having preferable anti-tumor effects.

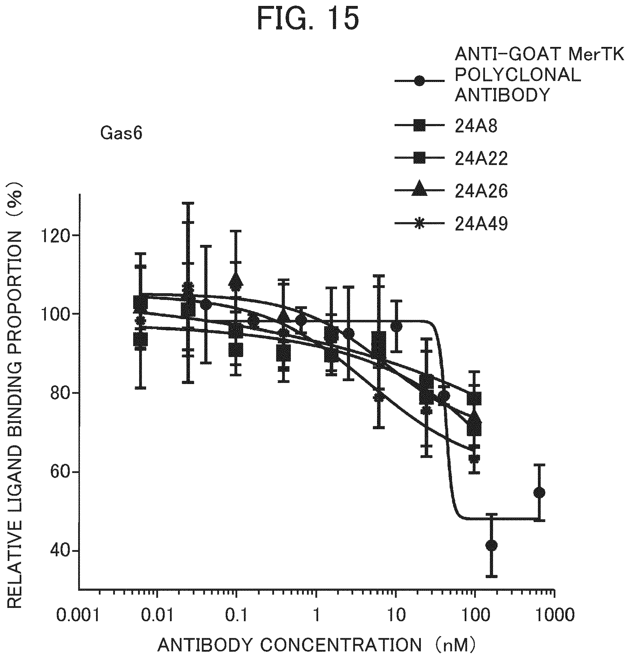

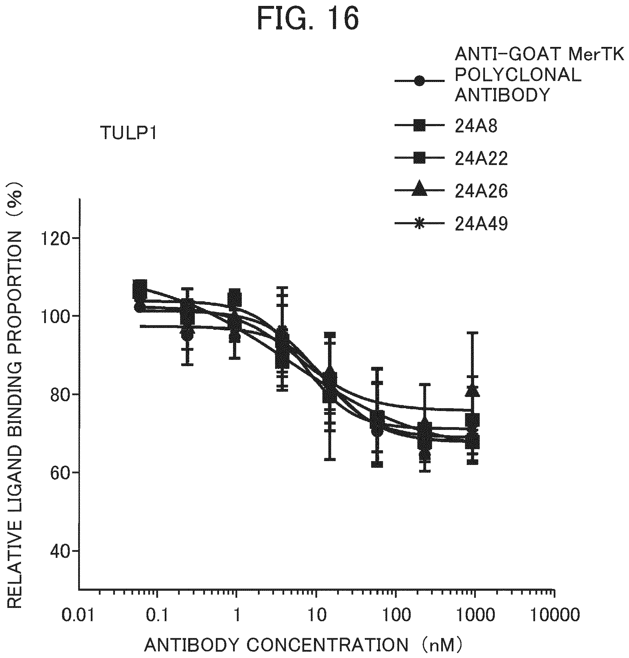

The inhibitory ability of ligand binding for many anti-MerTK antibodies was similarly evaluated. Figs. 15 and 16 show some of the results. Almost all antibodies inhibited both binding of Gas6 with MerTK and binding of TULP1 with MerTK. Thus, only "20A77" and "24A1" strongly inhibited binding of Gas6 with MerTK and did not inhibit binding of TULP1 with MerTK.

<Test 7: Study on ocular toxicity by in vivo ocular toxicity test>

In accordance with the results of "Test 6" above, in vivo ocular toxicity of the antibodies of the present invention was evaluated on the basis of the procedures indicated below.

The ocular toxicity test was carried out using Balb/c mice. The mice intravenously received "20A77_mIgG1", "24A1_mIgG1" or an isotype control antibody mIgG1 at a dose of 10 mg/kg at a frequency of twice a week. As a positive control of ocular toxicity, mice orally received a MerTK small molecule inhibitor UNC569 at a dose of 150 mg/kg for 10 days. At day 29 after the first administration, eyes were removed, fixed with a Karnovsky's fixative and then subjected to embedding in a resin, preparation of sections and toluidine blue staining (hereinafter also referred to as "TB staining") carried out by Applied Medical Research Laboratory. Increased TB-positive granules means progressed abnormality of the eye.

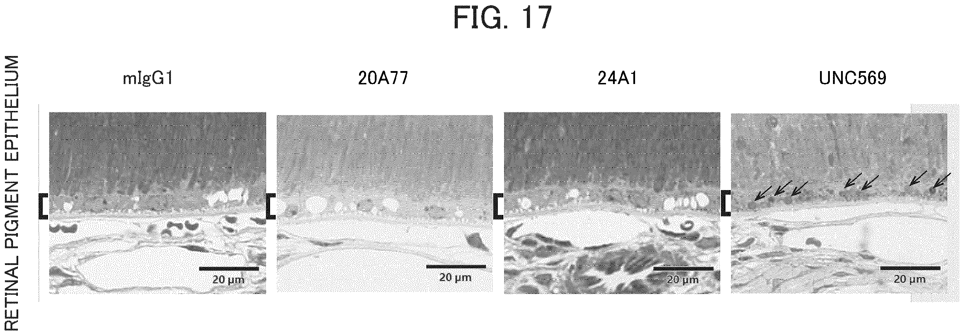

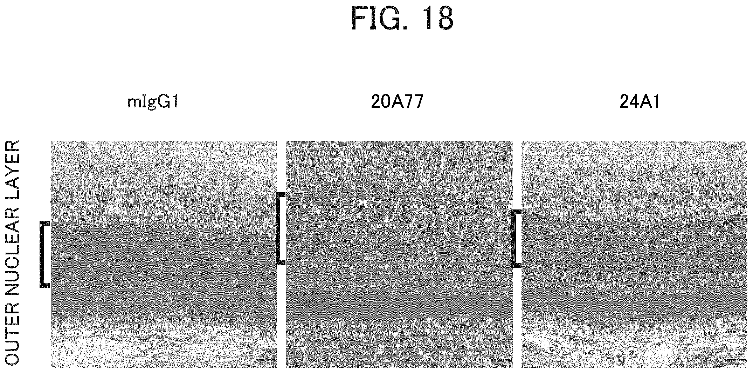

Figs. 17 and 18 show images of sections after TB staining. As shown in Fig. 17, the UNC569 administration group showed increased TB-positive granules (sites indicated with arrows) in the retinal pigment epithelium. Meanwhile, no increase in TB-positive granules was observed in the groups received "20A77_mIgG1" and "24A1_mIgG1".

As shown in Fig. 18, no shrinkage of the outer nuclear layer (abnormality recognized to correlate with deterioration of eyesight) was observed in any administration groups. The above results suggest that the antibodies of the present invention may be antibodies having low risk of ocular toxicity while providing anti-tumor effects.

<Test 8: Study on toxic dose by in vivo ocular toxicity test>

In accordance with the results of "Test 7" above, the antibody of the present invention was evaluated for toxic dose on the basis of the procedures indicated below.

The ocular toxicity test was carried out using Balb/c mice. The mice intravenously received a solvent or "m20A77_mIgG1" at a dose of 3 or 10 mg/kg at a frequency of twice a week. At day 30 after the first administration, eyes were removed, fixed with a Karnovsky's fixative and then subjected to embedding in a resin, preparation of sections and TB staining carried out by Applied Medical Research Laboratory.

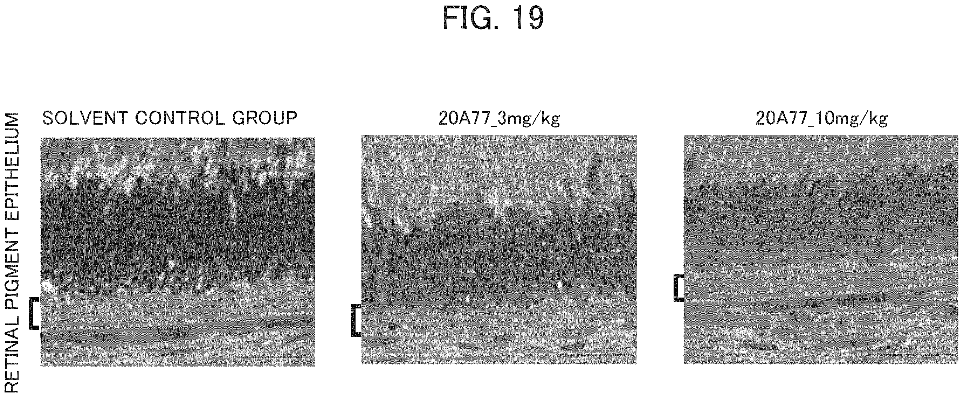

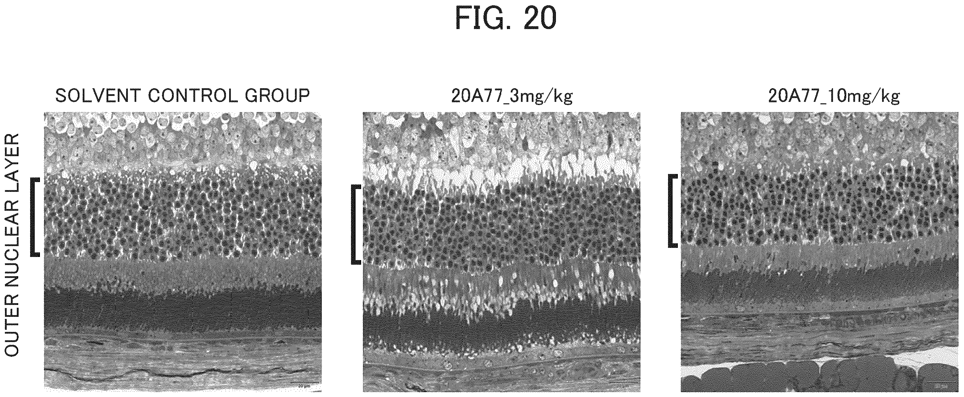

Figs. 19 and 20 show images of sections after TB staining. As shown in Fig. 19, no increase in TB-positive granules was observed in any administration groups. Meanwhile, as shown in Fig. 20, no abnormal shrinkage of the outer nuclear layer was observed in any of solvent control or administration groups of 3 mg/kg and 10 mg/kg.

<Test 9: Preparation of humanized antibodies>

Chimeric antibodies (hereinafter also referred to as "human chimeric antibodies") of mouse heavy and light chain variable regions and human IgG1 heavy and κ light chain constant regions were prepared for "m20A77" and "24A1". Human chimeric antibodies were prepared according to the same manner as in "(3) Preparation of recombinant mouse antibody and antibody sequence modification" above.

The obtained human chimeric antibodies were subjected to humanization of antibody sequence on the basis of the procedures indicated below. The sequences of antibody variable regions were humanized by CDR grafting. The humanized sequences were designed by referring to the method of Tsurushita et al. (2005, Design of humanized antibodies: From anti-Tac to Zenapax, Methods 36:69-83). Next, 3D molecular models of the mouse antibodies were prepared according to a conventional method. On the basis of the molecular models, residues considered to be important for CDR structure formation and residues considered to be essential for antigen reaction in the amino acid sequence of the framework region were estimated. In parallel to this process, sequences having high identity with the heavy chain variable regions and light chain variable regions of the antibodies were searched from the cDNA sequence database of human antibody heavy chain variable regions and light chain variable regions. Sequences were designed by connecting the sequence of the framework of the searched human antibody sequence and CDR sequences of each of the antibodies. Then, residues considered to be essential for CDR structure formation or antigen reaction were further grafted thereto, thereby designing the humanized antibody sequences "hz20A77" and "hz24A1" indicated in Table 10. The CDR sequences of the humanized antibody sequences are identical to those of the original mouse antibodies.

[Table 10]

|  | VH | CDRH 1 | CDRH 2 | CDRH 3 | VL | CDRL 1 | CDRL 2 | CDRL 3 |
|---|---|---|---|---|---|---|---|---|
| hz20A77 | SEQ ID NO:21 | SEQ ID NO:2 | SEQ ID NO:18 | SEQ ID NO:4 | SEQ ID NO:22 | SEQ ID NO:6 | SEQ ID NO:20 | SEQ ID NO:8 |
| hz24A1 | SEQ ID NO:23 | SEQ ID NO:10 | SEQ ID NO:11 | SEQ ID NO:12 | SEQ ID NO:24 | SEQ ID NO:14 | SEQ ID NO:15 | SEQ ID NO:16 |

The designed antibodies were prepared as indicated below. The DNA sequence encoding the amino acid sequence of the heavy chain variable region was linked to the α-lactalbumin signal peptide and cloned into pcDNA3.4 vector together with human IgG1 constant region having LALA sequence introduced therein (hereinafter also referred to as "hIgG1LALA") using "In-Fusion HD Cloning Kit" (Takara Bio Inc.). The DNA sequence encoding the amino acid sequence of the light chain variable region was linked to the α-lactalbumin signal peptide as the heavy chain variable region and cloned into pcDNA3.4 vector together with human Ig κ constant region using "In-Fusion HD Cloning Kit" (Takara Bio Inc.). Then antibodies were prepared according to the same manner as in "(3) Preparation of recombinant mouse antibody and antibody sequence modification" above.

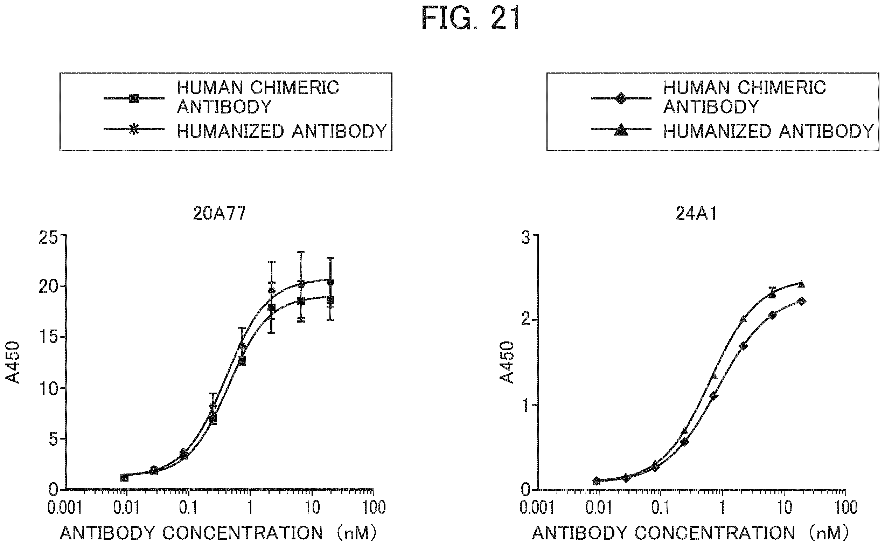

The prepared antibodies were checked for antigen binding activity by ELISA. Fig. 21 shows the results. As shown in Fig. 21, the obtained humanized antibodies ("hz20A77_hIgG1LALA" and "hz24A1_hIgG1LALA") showed reactivity similar to or higher than original human chimeric antibodies ("m20A77_hIgG1LALA" and "24A1_hIgG1LALA", respectively). These results show that the prepared humanized antibodies have sufficient reactivity for human MerTK.

## Claims

1. An antibody or fragment thereof that specifically binds to MerTK and has heavy chain and light chain CDRs 1-3 having amino acid sequences fulfilling all requirements in [Requirements A] indicated below:
   [Requirements A]

   the heavy chain CDR1 has an amino acid sequence given in SEQ ID NO: 2;
   the heavy chain CDR2 has an amino acid sequence given in SEQ ID NO: 3;
   the heavy chain CDR3 has an amino acid sequence given in SEQ ID NO: 4;
   the light chain CDR1 has an amino acid sequence given in SEQ ID NO: 6;
   the light chain CDR2 has an amino acid sequence given in SEQ ID NO: 7; and
   the light chain CDR3 has an amino acid sequence given in SEQ ID NO: 8.

2. The antibody or fragment thereof according to claim 1, further fulfilling all requirements in [Requirements B] indicated below:
   [Requirements B]

   the heavy chain variable region has an amino acid sequence given in SEQ ID NO: 1; and
   the light chain variable region has an amino acid sequence given in SEQ ID NO: 5.

3. An antibody or fragment thereof that specifically binds to MerTK and has heavy chain and light chain CDRs 1-3 having amino acid sequences fulfilling all requirements in [Requirements C] indicated below:
   [Requirements C]

   the heavy chain CDR1 has an amino acid sequence given in SEQ ID NO: 10;
   the heavy chain CDR2 has an amino acid sequence given in SEQ ID NO: 11;
   the heavy chain CDR3 has an amino acid sequence given in SEQ ID NO: 12;
   the light chain CDR1 has an amino acid sequence given in SEQ ID NO: 14;
   the light chain CDR2 has an amino acid sequence given in SEQ ID NO: 15; and
   the light chain CDR3 has an amino acid sequence given in SEQ ID NO: 16.

4. The antibody or fragment thereof according to claim 3, further fulfilling all requirements in either [Requirements D] or [Requirements E] indicated below:

   [Requirements D]

   the heavy chain variable region has an amino acid sequence given in SEQ ID NO: 9; and
   the light chain variable region has an amino acid sequence given in SEQ ID NO: 13;

   [Requirements E]

   the heavy chain variable region has an amino acid sequence given in SEQ ID NO: 23; and
   the light chain variable region has an amino acid sequence given in SEQ ID NO: 24.

5. An antibody or fragment thereof that specifically binds to MerTK and has heavy chain and light chain CDRs 1-3 having amino acid sequences fulfilling all requirements in [Requirements F] indicated below:
   [Requirements F]

   the heavy chain CDR1 has an amino acid sequence given in SEQ ID NO: 2;
   the heavy chain CDR2 has an amino acid sequence given in SEQ ID NO: 18;
   the heavy chain CDR3 has an amino acid sequence given in SEQ ID NO: 4;
   the light chain CDR1 has an amino acid sequence given in SEQ ID NO: 6;

the light chain CDR2 has an amino acid sequence given in SEQ ID NO: 20; and
the light chain CDR3 has an amino acid sequence given in SEQ ID NO: 8.

6. The antibody or fragment thereof according to claim 5, further fulfilling all requirements in either [Requirements G] or [Requirements H] indicated below:

[Requirements G]

the heavy chain variable region has an amino acid sequence given in SEQ ID NO: 17; and
the light chain variable region has an amino acid sequence given in SEQ ID NO: 19;

[Requirements H]

the heavy chain variable region has an amino acid sequence given in SEQ ID NO: 21; and
the light chain variable region has an amino acid sequence given in SEQ ID NO: 22.

7. An anti-tumor agent, comprising the antibody or fragment thereof according to any one of claims 1 to 6.

8. The anti-tumor agent according to claim 7, further comprising an immune checkpoint inhibitor.

# FIG. 1

# FIG. 2

20A77 VH

| CDR H1 | CDR H2 | CDR H3 |

QVQLQQPGTELVKPGASVKLSCRASGNTFTSYWMHWVKQRPGQGLEWIGNINPSNGGTNYNEKFKSKATLTVDKSSSTAYMQLSSLTSEDSAVYYCARLNYYGFDGFAFWGQGTLVTVSA

SG

20A77 VL

| CDR L1 | CDR L2 | CDR L3 |

DIKMTQSPSSMYASLGERVTITCKASQDINSYLSWFQQKPGKSPKTLIYRANRLVDGVPSRFSGSGSGQDYSLTISSLEYEDMGIYYCLQYDEFPFTFGSGTKLEIKR

SG

# FIG. 3

Legend:
- ◆ HUMAN MerTK
- ■ MOUSE MerTK
- ▲ CYNOMOLGUS MerTK
- ● RAT MerTK

20A77
CROSS-REACTIVITY

24A1
CROSS-REACTIVITY

FIG. 4

# FIG. 5

# FIG. 6

FIG. 7

# FIG. 8

FIG. 9

# FIG. 10

## FIG. 11

mIgG1 · m20A77 · 24A1 · αPD-1 · m20A77 + αPD-1 · 24A1 + αPD-1

TUMOR VOLUME (mm$^3$)

DAYS AFTER INOCULATION (DAY)

EP 4 417 625 A1

# FIG. 12

Legend:
- ●— mIgG1
- ■— m20A77
- ▲— 24A1
- ▼— α PD−1
- ◆— m20A77 + α PD−1
- ○— 24A1 + α PD−1

○ p=0.0171 vs α PD−1

p=0.0153 vs 24A1

Y-axis: AVERAGE TUMOR VOLUME $(mm^3)$

X-axis: DAYS AFTER INOCULATION (DAY)

29

# FIG. 13

# FIG. 14

# FIG. 15

# FIG. 16

TULP1

Legend:
- ● ANTI-GOAT MerTK POLYCLONAL ANTIBODY
- ■ 24A8
- ■ 24A22
- ▲ 24A26
- ✳ 24A49

Y-axis: RELATIVE LIGAND BINDING PROPORTION (%)
X-axis: ANTIBODY CONCENTRATION (nM)

# FIG. 17

UNC569

24A1

20A77

mIgG1

RETINAL PIGMENT EPITHELIUM

FIG. 18

mIgG1    20A77    24A1

OUTER NUCLEAR LAYER

# FIG. 19

EP 4 417 625 A1

RETINAL PIGMENT EPITHELIUM

SOLVENT CONTROL GROUP

20A77_3mg/kg

20A77_10mg/kg

## FIG. 20

20A77_10mg/kg

20A77_3mg/kg

SOLVENT CONTROL GROUP

OUTER NUCLEAR LAYER

# FIG. 21

EP 4 417 625 A1

**EP 4 417 625 A1**

## INTERNATIONAL SEARCH REPORT

International application No.

**PCT/JP2022/034801**

### A. CLASSIFICATION OF SUBJECT MATTER

*C07K 16/40*(2006.01)i; *A61K 39/395*(2006.01)i; *A61K 45/00*(2006.01)i; *A61P 35/00*(2006.01)i; *A61P 43/00*(2006.01)i; *C12N 15/13*(2006.01)i

FI:    C07K16/40 ZNA; A61P35/00; A61P43/00 121; A61K39/395 N; A61K39/395 T; A61K45/00; C12N15/13

According to International Patent Classification (IPC) or to both national classification and IPC

### B. FIELDS SEARCHED

Minimum documentation searched (classification system followed by classification symbols)

C07K16/00-16/46; A61K39/00-39/44; A61K45/00-45/08; A61P1/00-43/00; C12N15/00-15/90

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY/MEDLINE/EMBASE/BIOSIS (STN); UniProt/GeneSeq

### C. DOCUMENTS CONSIDERED TO BE RELEVANT

| Category* | Citation of document, with indication, where appropriate, of the relevant passages | Relevant to claim No. |
|---|---|---|
| A | WO 2021/119508 A1 (ALECTOR LLC) 17 June 2021 (2021-06-17)<br>claims, table 26 | 1-8 |
| A | WO 2020/076799 A1 (BRISTOL-MYERS SQUIBB COMPANY) 16 April 2020 (2020-04-16)<br>claims, p. 13, lines 14-29 | 1-8 |

☐ Further documents are listed in the continuation of Box C.    ☑ See patent family annex.

| | |
|---|---|
| \* Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **05 October 2022** | **08 November 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

International application No.

**PCT/JP2022/034801**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    The "form of an Annex C/ST.25 text file" is replaced with the "form of ST.26."

Form PCT/ISA/210 (continuation of first sheet) (January 2015)

## INTERNATIONAL SEARCH REPORT

| International application No. |
|---|
| **PCT/JP2022/034801** |

**Box No. III    Observations where unity of invention is lacking (Continuation of item 3 of first sheet)**

This International Searching Authority found multiple inventions in this international application, as follows:

Claims are classified into the following three inventions.

(Invention 1) Claims 1-2 and 5-6, and claims 7-8 referring to any of claims 1-2 and 5-6
    Claim 1 has the special technical feature of "having CDR, an amino acid sequence, represented by SEQ ID Nos: 2-8," and is thus classified as invention 1.
    Also, the invention in claim 5 shares, with the invention in claim 1, the common special technical feature of "having CDR, an amino acid sequence, represented by SEQ ID Nos: 2, 4, 6, and 8," and is thus classified as invention 1.
    The invention in claims 2 and 6-8 referring to any of claims 1 and 5 is dependent on claim 1 or 5, and is inventively associated with claims 1 and 5, and is thus classified as invention 1.

(Invention 2) Claims 3-4 and claims 7-8 referring to any of claims 3-4
    Claim 3 cannot be said to have a same or corresponding special technical feature with the invention classified as invention 1.
    Claim 3 is not dependent on claim 1, and is not substantially identical to or similarly closely related to any of the claims classified as invention 1.
    In addition, claim 3 has the special technical feature of "having CDR, an amino acid sequence, represented by SEQ ID Nos: 10-16," and is thus classified as invention 2.
    Also, the invention in claims 4 and 7-8 referring to claim 3 is dependent on claim 3, and is inventively associated with claim 3, and is thus classified as invention 2.

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
Information on patent family members

International application No.

**PCT/JP2022/034801**

| Patent document cited in search report | | Publication date (day/month/year) | Patent family member(s) | Publication date (day/month/year) |
|---|---|---|---|---|
| WO | 2021/119508 A1 | 17 June 2021 | (Family: none) | |
| WO | 2020/076799 A1 | 16 April 2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020076799 A

**Non-patent literature cited in the description**

- *Pharmacol Ther.,* 10 March 2021, vol. 225, 107822
- *Immunity.,* 11 February 2020, vol. 52 (2), 357-373.e9
- *Toxicol Pathol.,* 08 January 2018, vol. 46 (2), 193-201
- *Investigative Ophthalmology & Visual Science,* August 2001, vol. 42 (9
- *Neuron,* 20 December 2012, vol. 76 (6), 1123-32
- **KABAT et al.** Sequences of Proteins of Immunological Interests. U.S. Department of Health and Human Services, 1991
- **TSURUSHITA et al.** Design of humanized antibodies: From anti-Tac to Zenapax. *Methods,* 2005, vol. 36, 69-83